# EUROPEAN PATENT SPECIFICATION

(11) **EP 3 169 806 B1**
(45) Date of publication and mention of the grant of the patent: **01.05.2019**
(21) Application number: 15747666.4
(22) Date of filing: 17.07.2015
(51) Int. Cl.: C12Q 1/68, G06F 19/22

(54) **SYSTEMS AND METHODS FOR DETECTING STRUCTURAL VARIANTS**
SYSTEME UND VERFAHREN ZUR ERKENNUNG STRUKTURELLER VARIANTEN
SYSTÈMES ET PROCÉDÉS DE DÉTECTION DE VARIANTS STRUCTURAUX

(30) Priority: 18.07.2014 US 201462026300 P
(43) Date of publication of application: 24.05.2017
(73) Proprietor: Life Technologies Corporation, Carlsbad, CA 92008 (US)
(72) Inventor: GOTTIMUKKALA, Rajesh Kumar, Carlsbad, CA 92008 (US); HYLAND, Fiona C. Laird, Carlsbad, CA 92008 (US)
(74) Representative: Thermo Fisher Scientific- Life Sciences Solutions Group
(86) International application number: PCT/US2015/040951
(87) International publication number: WO 2016/011378

(56) References cited:
- US-A1- 2013 110 410
- EDGREN HENRIK ET AL: "Identification of fusion genes in breast cancer by paired-end RNA-sequencing", GENOME BIOLOGY, BIOMED CENTRAL LTD., LONDON, GB, vol. 12, no. 1, 19 January 2011 (2011-01-19), page R6, XP021091784, ISSN: 1465-6906, DOI: 10.1186/GB-2011-12-1-R6

## Description

### TECHNICAL FIELD

The present disclosure generally relates to the field of nucleic acid sequencing including systems and methods for detecting gene fusions.

### INTRODUCTION

Upon completion of the Human Genome Project, one focus of the sequencing industry has shifted to finding higher throughput and/or lower cost nucleic acid sequencing technologies, sometimes referred to as "next generation" sequencing (NGS) technologies. In making sequencing higher throughput and/or less expensive, one goal is to make the technology more accessible. This can be reached through the use of sequencing platforms and methods that provide sample preparation for samples of significant complexity, sequencing larger numbers of samples in parallel (for example through use of barcodes and multiplex analysis), and/or processing high volumes of information efficiently and completing the analysis in a timely manner. Various methods, such as, for example, sequencing by synthesis, sequencing by hybridization, and sequencing by ligation are evolving to meet these challenges.

Ultra-high throughput nucleic acid sequencing systems incorporating NGS technologies typically produce a large number of short sequence reads. Sequence processing methods should desirably assemble and/or map a large number of reads quickly and efficiently, such as to minimize use of computational resources. For example, data arising from sequencing of a mammalian genome can result in tens or hundreds of millions of reads that typically need to be assembled before they can be further analyzed to determine their biological, diagnostic and/or therapeutic relevance.

Exemplary applications of NGS technologies include, but are not limited to: genomic variant detection, such as insertions/deletions, copy number variations, single nucleotide polymorphisms, genomic resequencing, gene expression analysis, genomic profiling, and the like.

Structural variants, such as large scale deletions, insertions, inversions, genomic rearrangements, gene fusions, and the like, can be associated with various genetic disorders and cancers. Structure variants can often lead to significant disruptions in the production of proteins essential for the proper function of a cell. For example, genomic rearrangements and gene fusions can lead to mRNA coding for chimeric proteins, having a first part from one protein and a second part from another protein. Often, these chimeric proteins no longer function like either the first or second protein and can lead to disruption of regularity pathways. In cancer cells, the disrupted regulatory pathways may be involved in the regulation of apoptosis, cell growth, or the like and, as a result of the gene fusion, enable the cancer cells to grow unchecked.

From the foregoing it will be appreciated that a need exists for systems and methods that can detect gene fusions using nucleic acid sequencing data.

### DRAWINGS

For a more complete understanding of the principles disclosed herein, and the advantages thereof, reference is now made to the following descriptions taken in conjunction with the accompanying drawings, in which:
Figure 1 is a block diagram that illustrates an exemplary computer system, in accordance with various embodiments.
Figure 2 is a schematic diagram of an exemplary system for reconstructing a nucleic acid sequence, in accordance with various embodiments.
Figure 3 is a schematic diagram of an exemplary genetic analysis system, in accordance with various embodiments.
Figure 4 is a diagram illustrating an exemplary gene fusion, in accordance with various embodiments.
Figure 5 is a flow diagram illustrating an exemplary method of detecting gene fusions, in accordance with various embodiments.
Figure 6 is a diagram illustrating an exemplary synthetic control, in accordance with various embodiments.

It is to be understood that the figures are not necessarily drawn to scale, nor are the objects in the figures necessarily drawn to scale in relationship to one another. The figures are depictions that are intended to bring clarity and understanding to various embodiments of apparatuses, systems, and methods disclosed herein. Wherever possible, the same reference numbers will be used throughout the drawings to refer to the same or like parts. Moreover, it should be appreciated that the drawings are not intended to limit the scope of the present teachings in any way.

### DESCRIPTION OF VARIOUS EMBODIMENTS

Embodiments of systems and methods for detecting gene fusions are described and illustrated herein.

The section headings used herein are for organizational purposes only and are not to be construed as limiting the described subject matter in any way.

In this detailed description of the various embodiments, for purposes of explanation, numerous specific details are set forth to provide a thorough understanding of the embodiments disclosed. One skilled in the art will appreciate, however, that these various embodiments may be practiced with or without these specific details. In other instances, structures and devices are shown in block diagram form. Furthermore, one skilled in the art can readily appreciate that the specific sequences in which methods are presented and performed are illustrative and it is contemplated that the sequences can be varied and still remain within the scope of the various embodiments disclosed herein.

Unless described otherwise, all technical and scientific terms used herein have a meaning as is commonly understood by one of ordinary skill in the art to which the various embodiments described herein belongs.

It will be appreciated that there is an implied "about" prior to the temperatures, concentrations, times, number of bases, coverage, etc. discussed in the present teachings, such that slight and insubstantial deviations are within the scope of the present teachings. In this application, the use of the singular includes the plural unless specifically stated otherwise. Also, the use of "comprise", "comprises", "comprising", "contain", "contains", "containing", "include", "includes", and "including" are not intended to be limiting. It is to be understood that both the foregoing general description and the following detailed description are exemplary and explanatory only and are not restrictive of the present teachings.

As used herein, "a" or "an" also may refer to "at least one" or "one or more." Also, the use of "or" is inclusive, such that the phrase "A or B" is true when "A" is true, "B" is true, or both "A" and "B" are true.

Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used, for example, for nucleic acid purification and preparation, chemical analysis, recombinant nucleic acid, and oligonucleotide synthesis. Enzymatic reactions and purification techniques are performed according to manufacturer's specifications or as commonly accomplished in the art or as described herein. The techniques and procedures described herein are generally performed according to conventional methods well known in the art and as described in various general and more specific references that are cited and discussed throughout the instant specification. *See, e.g*., Sambrook et al., Molecular Cloning: A Laboratory Manual (Third ed., Cold Spring Harbor Laboratory Press, Cold Spring Harbor, N.Y. 2000). The nomenclatures utilized in connection with, and the laboratory procedures and techniques described herein are those well known and commonly used in the art.

In various embodiments, a "system" sets forth a set of components, real or abstract, comprising a whole where each component interacts with or is related to at least one other component within the whole.

In various embodiments, a "biomolecule" may refer to any molecule that is produced by a biological organism, including large polymeric molecules such as proteins, polysaccharides, lipids, and nucleic acids (DNA and RNA) as well as small molecules such as primary metabolites, secondary metabolites, and other natural products.

In various embodiments, the phrase "next generation sequencing" or NGS refers to sequencing technologies having increased throughput as compared to traditional Sanger- and capillary electrophoresis-based approaches, for example with the ability to generate hundreds of thousands of relatively small sequence reads at a time. Some examples of next generation sequencing techniques include, but are not limited to, sequencing by synthesis, sequencing by ligation, and sequencing by hybridization. More specifically, the Personal Genome Machine (PGM) and Proton of Life Technologies Corp. provides massively parallel sequencing with enhanced accuracy. The PGM and Proton Systems and associated workflows, protocols, chemistries, etc. are described in more detail in U.S. Patent Application Publication No. 2009/0127589 and No. 2009/0026082.

In various embodiments, the phrase "sequencing run" refers to any step or portion of a sequencing experiment performed to determine some information relating to at least one biomolecule (e.g., nucleic acid molecule).

In various embodiments, the phase "base space" refers to a representation of a sequence of nucleotides. The phase "flow space" refers to a representation of an incorporation event or non-incorporation event for a particular nucleotide flow. For example, flow space can be a series of values representing a nucleotide incorporation events (such as a one, "1") or a non-incorporation event (such as a zero, "0") for that particular nucleotide flow. Nucleotide flows having a non-incorporation event can be referred to as empty flows. Nucleotide flows having a nucleotide incorporation event can be referred to as positive flows. It should be understood that zeros and ones are convenient representations of a non-incorporation event and a nucleotide incorporation event; however, any other symbol or designation could be used alternatively to represent and/or identify incorporation and non-incorporation events. In particular, when multiple nucleotides are incorporated at a given position, such as for a homopolymer stretch, the value can be proportional to the number of nucleotide incorporation events and thus the length of the homopolymer stretch (e.g., greater than one).

In various embodiments, DNA (deoxyribonucleic acid) may be referred to as a chain of nucleotides consisting of 4 types of nucleotides; A (adenine), T (thymine), C (cytosine), and G (guanine), and that RNA (ribonucleic acid) is comprised of 4 types of nucleotides; A, U (uracil), G, and C. Certain pairs of nucleotides specifically bind to one another in a complementary fashion (called complementary base pairing). That is, adenine (A) pairs with thymine (T) (in the case of RNA, however, adenine (A) pairs with uracil (U)), and cytosine (C) pairs with guanine (G). When a first nucleic acid strand binds to a second nucleic acid strand made up of nucleotides that are complementary to those in the first strand, the two strands bind to form a double strand. In various embodiments, "nucleic acid sequencing data," "nucleic acid sequencing information," "nucleic acid sequence," "genomic sequence," "genetic sequence," or "fragment sequence," or "nucleic acid sequencing read" denotes any information or data that is indicative of the order of the nucleotide bases (e.g., adenine, guanine, cytosine, and thymine/uracil) in a molecule (e.g., whole genome, whole transcriptome, exome, oligonucleotide, polynucleotide, fragment, etc.) of DNA or RNA. It should be understood that the present teachings contemplate sequence information obtained using all available varieties of techniques, platforms or technologies, including, but not limited to: capillary electrophoresis, microarrays, ligation-based systems, polymerase-based systems (such as Illumina HiSeq, MiSeq, and Genome Analyzer), hybridization-based systems, direct or indirect nucleotide identification systems, pyrosequencing (such as 454 Life Science GS FLX and GS Junior), ion- or pH-based detection systems (such as Ion Torrent), electronic signature-based systems (such as Oxford Nanopore GridION and MinION), etc.

In various embodiments, a "polynucleotide", "nucleic acid", or "oligonucleotide" refers to a linear polymer of nucleosides (including deoxyribonucleosides, ribonucleosides, or analogs thereof) joined by internucleosidic linkages. Typically, a polynucleotide comprises at least three nucleotides. Usually oligonucleotides range in size from a few monomeric units, e.g. 3-4, to several hundreds of monomeric units. Whenever a polynucleotide such as an oligonucleotide is represented by a sequence of letters, such as "ATGCCTG," it will be understood that the nucleotides are in 5'-3' order from left to right and that "A" denotes deoxyadenosine, "C" denotes deoxycytidine, "G" denotes deoxyguanosine, and "T" denotes thymidine, unless otherwise noted. The letters A, C, G, and T may be used to refer to the bases themselves, to nucleosides, or to nucleotides comprising the bases, as is standard in the art.

In various embodiments, a "structural variant" refers to a variation in the structure of a chromosome. Structural variants can include deletions, duplications, copy-number variants, insertions, gene fusions, inversions and translocations. Many of structural variants are associated with genetic diseases, however more are not.

### MULTIPLEX AMPLIFICATION METHODS:

In various embodiments, target nucleic acids generated by the amplification of multiple target-specific sequences from a population of nucleic acid molecules can be sequenced. In some exemplary embodiments, the amplification can include hybridizing one or more target-specific primer pairs to the target sequence, extending a first primer of the primer pair, denaturing the extended first primer product from the population of nucleic acid molecules, hybridizing to the extended first primer product the second primer of the primer pair, extending the second primer to form a double stranded product, and digesting the target-specific primer pair away from the double stranded product to generate a plurality of amplified target sequences. In some embodiments, the amplified target sequences can be ligated to one or more adapters. In some embodiments, the adapters can include one or more nucleotide barcodes or tagging sequences. In some embodiments, the amplified target sequences once ligated to an adapter can undergo a nick translation reaction and/or further amplification to generate a library of adapter-ligated amplified target sequences. Exemplary methods of multiplex amplification are described in U.S. Application No. 13/458,739 filed November 12, 2012 and titled "Methods and Compositions for Multiplex PCR", now published as US 2012/0295819 A1.

In various exemplary embodiments, a method of performing multiplex PCR amplification includes contacting a plurality of target-specific primer pairs having a forward and reverse primer, with a population of target sequences to form a plurality of template/primer duplexes; adding a DNA polymerase and a mixture of dNTPs to the plurality of template/primer duplexes for sufficient time and at sufficient temperature to extend either (or both) the forward or reverse primer in each target-specific primer pair via template-dependent synthesis thereby generating a plurality of extended primer product/template duplexes; denaturing the extended primer product/template duplexes; annealing to the extended primer product the complementary primer from the target-specific primer pair; and extending the annealed primer in the presence of a DNA polymerase and dNTPs to form a plurality of target-specific double-stranded nucleic acid molecules.

### COMPUTER-IMPLEMENTED SYSTEM

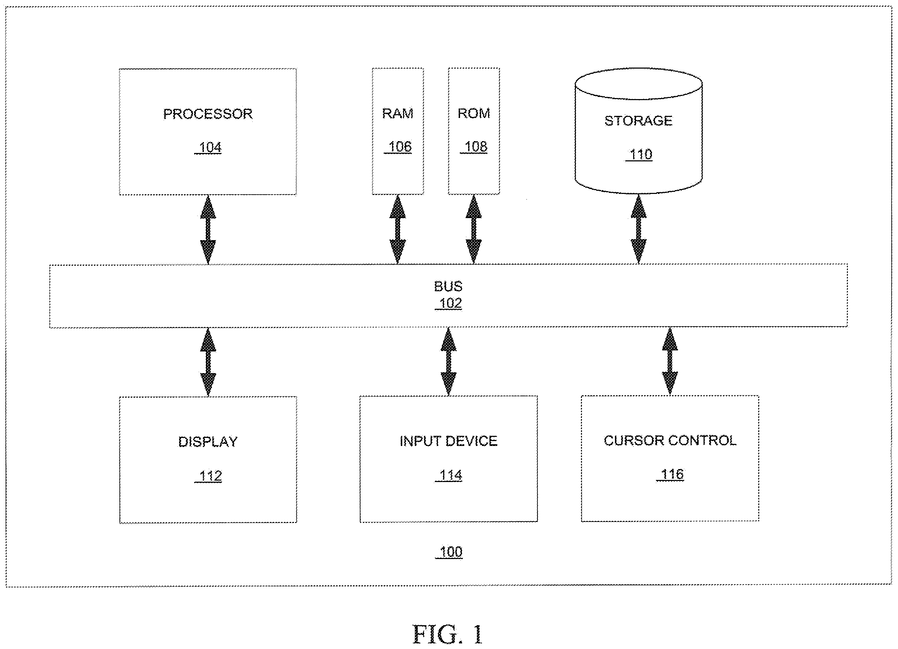

Figure 1 is a block diagram that illustrates an exemplary computer system 100, upon which embodiments of the present disclosure may be implemented. In various embodiments, computer system 100 can include a bus 102 or other communication mechanism for communicating information, and a processor 104 coupled with bus 102 for processing information. In various embodiments, computer system 100 can also include a memory 106, which can be a random access memory (RAM) or other dynamic storage device, coupled to bus 102 for determining base calls, and instructions to be executed by processor 104. Memory 106 also can be used for storing temporary variables or other intermediate information during execution of instructions to be executed by processor 104. In various embodiments, computer system 100 can further include a read only memory (ROM) 108 or other static storage device coupled to bus 102 for storing static information and instructions for processor 104. A storage device 110, such as a magnetic disk or optical disk, can be provided and coupled to bus 102 for storing information and instructions.

In various embodiments, computer system 100 can be coupled via bus 102 to a display 112, such as a cathode ray tube (CRT), a liquid crystal display (LCD), or other display or mechanism, for displaying or otherwise providing information to a computer user. An input device 114, including alphanumeric and other keys, can be coupled to bus 102 for communicating information and command selections to processor 104. Another type of user input device is a cursor control 116, such as a mouse, a trackball, cursor direction keys, and the like. for communicating direction information and command selections to processor 104 and for controlling cursor movement on display 112. This input device typically has two degrees of freedom in two axes, a first axis (i.e., x) and a second axis (i.e., y), that allows the device to specify positions in a plane.

In various exemplary embodiments, a computer system 100 can perform at least portions of the methods in accordance with the present disclosure. Consistent with certain implementations of the present teachings, results can be provided by computer system 100 in response to processor 104 executing one or more sequences of one or more instructions contained in memory 106. Such instructions can be read into memory 106 from another computer-readable medium, such as storage device 110. Execution of the sequences of instructions contained in memory 106 can cause processor 104 to perform the processes described herein. Alternatively hard-wired circuitry can be used in place of or in combination with software instructions to implement the present teachings. Thus implementations of the present teachings are not limited to any specific combination of hardware circuitry and software.

In various embodiments, the term "computer-readable medium" as used herein refers to any media that participates in providing instructions to processor 104 for execution. Such a medium can take many forms, including but not limited to, non-volatile media, volatile media, and transmission media. Examples of non-volatile media can include, but are not limited to, optical or magnetic disks, such as storage device 110. Examples of volatile media can include, but are not limited to, dynamic memory, such as memory 106. Examples of transmission media can include, but are not limited to, coaxial cables, copper wire, and fiber optics, including the wires that comprise bus 102.

Common forms of non-transitory computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, or any other tangible medium from which a computer can read.

In accordance with various embodiments, instructions configured to be executed by a processor to perform a method are stored on a computer-readable medium. The computer-readable medium can be a device that stores digital information. For example, a computer-readable medium includes a compact disc read-only memory (CD-ROM) as is known in the art for storing software. The computer-readable medium is accessed by a processor suitable for executing instructions configured to be executed.

### NUCLEIC ACID SEQUENCING PLATFORMS

Nucleic acid sequence data can be generated using various techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, electronic signature-based systems, etc.

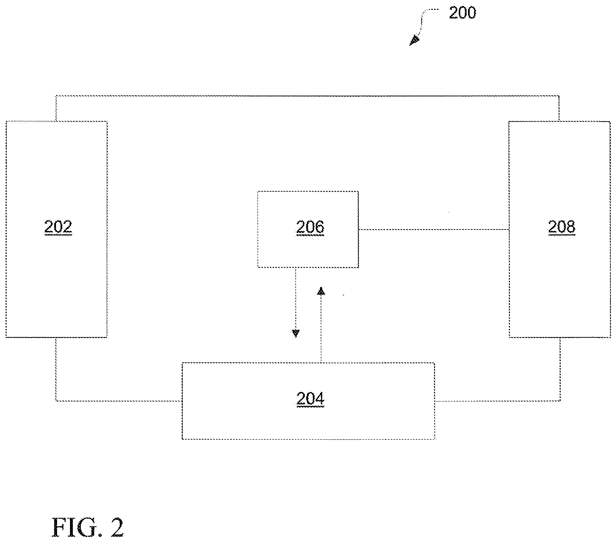

Various embodiments of nucleic acid sequencing platforms, such as a nucleic acid sequencer, can include components as displayed in the block diagram of Figure 2. According to various embodiments, sequencing instrument 200 can include a fluidic delivery and control unit 202, a sample processing unit 204, a signal detection unit 206, and a data acquisition, analysis and control unit 208. Various embodiments of instrumentation, reagents, libraries and methods used for next generation sequencing are described in U.S. Patent Application Publication No. 2009/0127589 and No. 2009/0026082. Various embodiments of instrument 200 can provide for automated sequencing that can be used to gather sequence information from a plurality of sequences in parallel, such as substantially simultaneously.

In various embodiments, the fluidics delivery and control unit 202 can include reagent delivery system. The reagent delivery system can include a reagent reservoir for the storage of various reagents. The reagents can include RNA-based primers, forward/reverse DNA primers, oligonucleotide mixtures for ligation sequencing, nucleotide mixtures for sequencing-by-synthesis, optional ECC oligonucleotide mixtures, buffers, wash reagents, blocking reagent, stripping reagents, and the like. The implemented primers can be designed for particular targets or may be universal primers. Additionally, the reagent delivery system can include a pipetting system or a continuous flow system which connects the sample processing unit with the reagent reservoir.

In various embodiments, the sample processing unit 204 can include a sample chamber, such as flow cell, a substrate, a micro-array, a multi-well tray, a through hole, or the like. The sample processing unit 204 can include multiple lanes, multiple channels, multiple wells, or other means of processing multiple sample sets substantially simultaneously. Additionally, the sample processing unit can include multiple sample chambers to enable processing of multiple runs simultaneously. In particular embodiments, the system can perform signal detection on one sample chamber while substantially simultaneously processing another sample chamber. Additionally, the sample processing unit can include an automation system for moving or manipulating the sample chamber.

In various embodiments, the signal detection unit 206 can include an imaging or detection sensor. For example, the imaging or detection sensor can include a CCD, a CMOS, an ion or chemical sensor, such as an ion sensitive layer overlying a CMOS or FET, a current or voltage detector, or the like. The signal detection unit 206 can include an excitation system to cause a probe, such as a fluorescent dye, to emit a signal. The excitation system can include an illumination source, such as arc lamp, a laser, a light emitting diode (LED), or the like. In particular embodiments, the signal detection unit 206 can include optics for the transmission of light from an illumination source to the sample or from the sample to the imaging or detection sensor. Alternatively, the signal detection unit 206 may provide for electronic or non-photon based methods for detection and consequently not include an illumination source. In various embodiments, electronic-based signal detection may occur when a detectable signal or species is produced during a sequencing reaction. For example, a signal can be produced by the interaction of a released byproduct or moiety, such as a released ion, such as a hydrogen ion, interacting with an ion or chemical sensitive layer. In other embodiments a detectable signal may arise as a result of an enzymatic cascade such as used in pyrosequencing (see, for example, U.S. Patent Application Publication No. 2009/0325145) where pyrophosphate is generated through base incorporation by a polymerase which further reacts with ATP sulfurylase to generate ATP in the presence of adenosine 5' phosphosulfate wherein the ATP generated may be consumed in a luciferase mediated reaction to generate a chemiluminescent signal. In another example, changes in an electrical current can be detected as a nucleic acid passes through a nanopore without the need for an illumination source.

In various embodiments, a data acquisition analysis and control unit 208 can monitor various system parameters. The system parameters can include temperature of various portions of instrument 200, such as sample processing unit or reagent reservoirs, volumes of various reagents, the status of various system subcomponents, such as a manipulator, a stepper motor, a pump, or the like, or any combination thereof.

It will be appreciated by one skilled in the art that various embodiments of instrument 200 can be used to practice variety of sequencing methods including ligation-based methods, sequencing by synthesis, single molecule methods, nanopore sequencing, and other sequencing techniques.

In various embodiments, the sequencing instrument 200 can determine the sequence of a nucleic acid, such as a polynucleotide or an oligonucleotide. The nucleic acid can include DNA or RNA, and can be single stranded, such as ssDNA and RNA, or double stranded, such as dsDNA or a RNA/cDNA pair. In various embodiments, the nucleic acid can include or be derived from a fragment library, a mate pair library, a ChIP fragment, or the like. In particular embodiments, the sequencing instrument 200 can obtain the sequence information from a single nucleic acid molecule or from a group of substantially identical nucleic acid molecules.

In various embodiments, sequencing instrument 200 can output nucleic acid sequencing read data in a variety of different output data file types/formats, including, but not limited to: *.fasta, *.csfasta, *seq.txt, *qseq.txt, *.fastq, *.sff, *prb.txt, *.sms, *.srs, *.bam, and/or *.qv.

### SYSTEM AND METHODS FOR IDENTIFYING SEQUENCE VARIATION

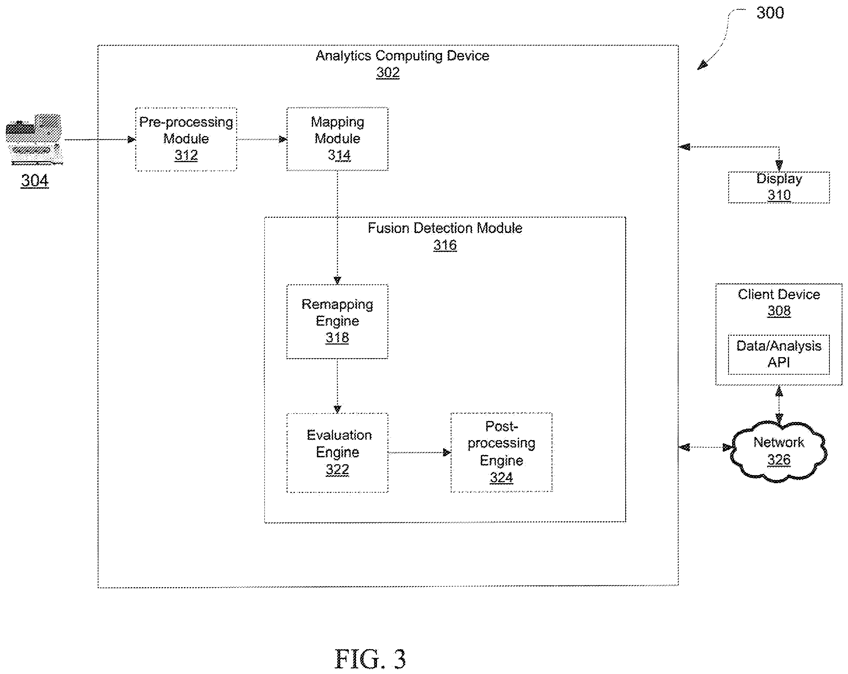

Figure 3 is a schematic diagram of a system for identifying variants, in accordance with various embodiments.

As depicted herein, variant analysis system 300 can include a nucleic acid sequence analysis device 304 (e.g., a nucleic acid sequencer, real-time PCR instrument, digital PCR (dPCR) instrument, quantitative PCR (qPCR) instrument, microarray scanner, and the like), an analytics computing device 302, and a display 310 and/or a client device terminal 308.

In various embodiments, the analytics computing device 302 can be communicatively connected to the nucleic acid sequence analysis device 304, and client device terminal 308 via a network connection 326 that can be either a wired physical network connection (e.g., Internet, LAN, WAN, VPN, etc.) or a wireless network connection (e.g., Wi-Fi, WLAN, etc.).

In various embodiments, the analytics computing device 302 can be a workstation, mainframe computer, distributed computing node (such as, part of a "cloud computing" or distributed networking system), personal computer, mobile device, server, or the like. In various embodiments, the nucleic acid sequence analysis device 304 can be a nucleic acid sequencer, real-time PCR instrument, digital PCR (dPCR) instrument, quantitative PCR (qPCR) instrument, microarray scanner, or the like. It should be understood, however, that the nucleic acid sequence analysis device 304 can be any type of instrument that can generate nucleic acid sequence data from samples (e.g., containing nucleic acid molecules) obtained from an individual.

The analytics computing device 302 can be configured to host an optional pre-processing module 312, a mapping module 314, and a fusion detection module 316.

Pre-processing module 312 can be configured to receive from the nucleic acid sequence analysis device 304 and perform processing steps, such as conversion from f space to base space, color space to base space, or from flow space to base space, determining call quality values, preparing the read data for use by the mapping module 314, and the like. Quality values can be a per base or per flow estimate of the accuracy of the sequencing.

The mapping module 314 can be configured to align (i.e., map) a nucleic acid sequence read to a reference sequence. In some examples, the length of the sequence read will be substantially less than the length of the reference sequence. In reference sequence mapping or alignment, sequence reads are assembled against an existing backbone sequence (e.g., reference sequence, etc.) to build a sequence that is similar but not necessarily identical to the backbone sequence. Once a backbone sequence is found for an organism, comparative sequencing or re-sequencing can be used to characterize the genetic diversity within the organism's species or between closely related species. In various embodiments, the reference sequence can be a whole/partial genome, whole/partial exome, and the like.

In various embodiments, the sequence read and reference sequence can be represented as a sequence of nucleotide base symbols in a base space. In various embodiments, the sequence read and reference sequence can be represented as one or more colors in a color space. In various embodiments, the sequence read and reference sequence can be represented as nucleotide base symbols with signal or numerical quantitation components in a flow space.

In various embodiments, the alignment of the sequence read and reference sequence can include a limited number of mismatches between the bases that comprise the sequence read and the bases that comprise the reference sequence. In some examples, the sequence read can be aligned to a portion of the reference sequence in order to minimize the number of mismatches between the sequence read and the reference sequence.

The fusion detection module 316 can include a remapping engine 318, an evaluation engine 322, and an optional post processing engine 324. In various embodiments, fusion detection module 316 can be in communication with the mapping module 314. That is, fusion detection module 316 can request and receive data and information (through, e.g., data streams, data files, text files, and the like.) from mapping module 314.

The remapping engine 318 can be configured to receive mapped reads from the mapping module 314, and identify reads that are partially aligned (partially mapped). For example, the reads can be aligned at the beginning or the end of the read, and may be aligned not greater than 60% of the read length, not greater than 50% of the read length, or not greater than 40% of the read length. Additionally, the remapping engine 318 can split the reads separating the mapped portion from the unmapped portion to generate a set of read fragments. The read fragments can be mapped to the reference genome, and a set of candidate fusions can be generated by combining the locations where the first fragment and the second fragment of a read are mapped.

Evaluation engine 322 can be configured to receive evidence from the remapping engine 318. The evaluation engine 322 can obtain counts of the reads supporting candidate fusions. Optionally, the evaluation engine 322 can use annotations of the genomic loci to consolidate and filter the candidate fusions. Based on the evidence received from the remapping engine 318, the evaluation engine can classify the variant and determine a confidence value. In various embodiments, the evaluation engine 322 can filter the candidate fusions based on the number of counts, annotation of both loci of a candidate fusion with gene names, annotation of at least one locus with an exon number, and the like.

Post processing engine 324 can be configured to receive the gene fusions identified by the evaluation engine 318 and perform additional processing steps, such as selecting and formatting the data for display on display 310 or use by client device 308.

Client device 308 can be a thin client or thick client computing device. In various embodiments, client device 308 can have a web browser (e.g., INTERNET EXPLORER™, FIREFOX™, SAFARI™, etc) that can be used to communicate information to and/or control the operation of the pre-processing module 312, mapping module 314, remapping engine 318, evaluation engine 322, and post processing engine 324 using a browser to control their function. The web browser may execute software (e.g., web applications, applets, and the like) configured to operate nucleic acid sequence analysis device 304. For example, the client device 308 can be used to configure the operating parameters (e.g., match scoring parameters, annotations parameters, filtering parameters, data security and retention parameters, etc.) of the various modules, depending on the requirements of the particular application. Similarly, client device 308 can also be configured to display the results of the analysis performed by the fusion detection module 316 and the nucleic acid sequencer 304. Client device 308 may execute any other suitable software (e.g., stand-alone applications) configured to operate nucleic acid sequence analysis device 304.

It should be understood that the various data stores disclosed as part of system 300 can represent hardware-based storage devices (e.g., hard drive, flash memory, RAM, ROM, network attached storage, etc.) or instantiations of a database stored on a standalone or networked computing device(s) (e.g., virtual data storage). It should also be appreciated that the various data stores and modules or engines shown as being part of the system 300 can be combined or collapsed into a single module, engine, and/or data store, or expanded in multiple modules, engines, and/or data stores depending on the configuration of the particular application or system architecture implemented. Moreover, in various embodiments, the system 300 can comprise additional modules, engines, components or data stores as needed by the particular application or system architecture.

In various embodiments, the system 300 can be configured to process the nucleic acid reads in color space. In various embodiments, system 300 can be configured to process the nucleic acid reads in base space. In various embodiments, system 300 can be configured to process the nucleic acid sequence reads in flow space. It should be understood to one of ordinary skill in the art, however, that the system 300 disclosed herein can process or analyze nucleic acid sequence data in any schema or format as long as the schema or format can convey the base identity and position of the nucleic acid sequence.

In various embodiments, system 300 may be used to identify candidates for gene fusions. For example, a gene may have experienced a fusion event, such a translocation, interstitial deletion, chromosomal inversion, and the like. The gene resulting from this combination may introduce complexities to the sequencing analysis.

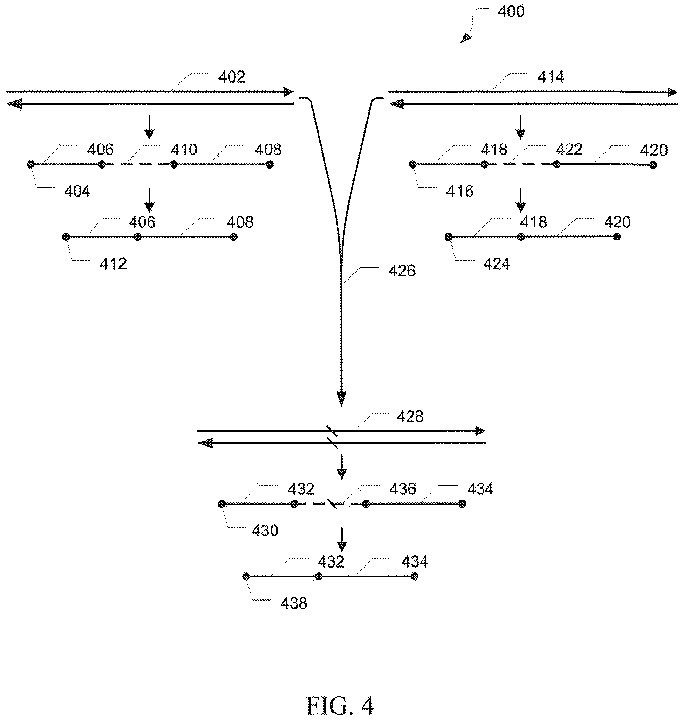

Figure 4 is a diagram showing an exemplary gene fusion 400. Prior to a gene fusion event, exemplary gene 402 can be transcribed into an RNA 404 containing exons 406 and 408 and an intron 410. Splicing of the RNA 404 can remove intron 410 and produce an mRNA 412 including exons 406 and 408.

Similarly, exemplary gene 414 can be transcribed into an RNA 416 containing exons 418 and 420 and an intron 422. Splicing of the RNA 416 can remove intron 422 and produce an mRNA 424 including exons 418 and 420. A fusion event 426 can combine portions of gene 402 and gene 414 into fusion gene 428. Fusion gene 428 can be transcribed into an RNA 430 containing exons 432 and 434 and an intron 436. In various embodiments, exon 432 can correspond to exon 406 of gene 402, and exon 434 can correspond to exon 420 of gene 414. Splicing of the RNA 430 can remove intron 436 and produce an mRNA 438 including exons 432 and 434. Presence of mRNA 438 in a sample can be indicative of the presence of fusion gene 428. Additionally, the fusion event 426 that produces fusion gene 428 can disrupt the production of mRNA 412 and 424, as well as any proteins encoded by mRNA 412 and 424.

In various embodiments, detection techniques may aid in the sequencing of genes that have experienced a fusion event. For example, the detection of particular mapping conditions, such as, for example, partially aligned reads for a beginning or end of a read, may indicate a gene that has experienced a fusion event. Based on the detection of such conditions, additional processing may be performed to identify the fused gene portions, or candidate gene portions, involved in the fusion event.

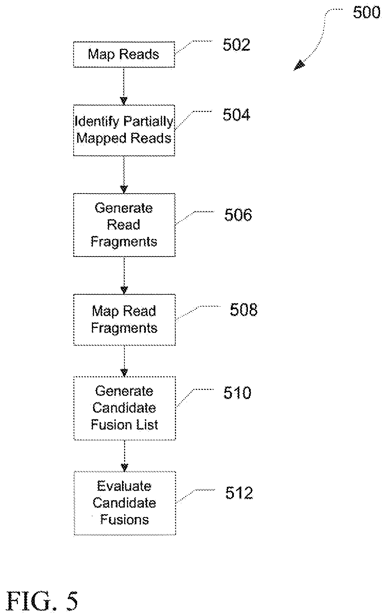

Figure 5 is a flow diagram illustrating an exemplary method 500 of detecting gene fusions. At 502, reads can be mapped to a reference, such as a reference genome or transcriptome. For example, a plurality of amplicons may be generated in the presence of a primer pool. In various embodiments, the reads can be generated by sequencing amplicons generated from a multiplex amplification. The amplification can include a first set of primers corresponding to 3' end of a first plurality of exons and a second set of primers corresponding to a 5' end of a second plurality of exons. In various embodiments, amplicons can be generated from a plurality of known exon-exon junctions in a gene. Additionally, amplicons can be generated from gene fusions, where an exon from a first gene is joined to a portion of a second gene, as discussed herein.

In various embodiments, the amplicons may undergo a variety of sequencing reactions that result in signals emission such that a plurality of reads may be generated based on the detected signals. The sequence reads and reference sequence can be represented as a sequence of nucleotide base symbols in a base space, one or more colors in a color space, nucleotide base symbols with signal or numerical quantitation components in a flow space, or as any other suitable representation.

In various embodiments, mapping may include aligning a nucleic acid sequence read to a reference genome. In some examples, the length of the sequence read will be substantially less than the length of the reference genome. In reference sequence mapping or alignment, sequence reads may be assembled against an existing backbone sequence (e.g., reference sequence) to build a sequence that is similar but not necessarily identical to the backbone sequence.

In various embodiments, the alignment of the sequence read and reference genome can include a limited number of mismatches between the bases that comprise the sequence read and the bases that comprise the reference sequence. In some examples, the sequence read can be aligned to a portion of the reference sequence in order to minimize the number of mismatches between the sequence read and the reference sequence.

At 504, a subset of reads can be identified that are partially mapped to the reference genome. Specifically, the reads can have a mapped portion and an unmapped portion. The mapped portion may be near the beginning of the read with the unmapped portion near the end of the read, or the unmapped portion can be near the beginning of the read and the mapped portion can be near the end of the read. For example, the mapped portion may be within a threshold distance (e.g., threshold absolute number or percentage number of bases) from the beginning or end of the read. In various embodiments, the mapped portion can be not greater than 50% of the read length, such as not greater than 50% of the read length, not greater than 40% of the read length, or any other suitable percentage. The mapped portion may be mapped within a threshold distance from the reference sequence, such that the mapped portion includes a threshold number of mismatches with the reference sequence.

At 506, the reads of the subset can be split into the mapped portion and the unmapped portion, such that a read of the subset generates two read fragments. For example, the reads may be soft clipped such that a first read fragment includes the mapped portion of the read and a second read fragment includes the unmapped portion of the read. In an exemplary embodiment, each read fragment may be associated with a key (e.g., R1, R2, R3, and the like) identifying the partially mapped read from which the fragment was generated.

At 508, the read fragments generated from the partially mapped reads can be aligned to the reference genome. In an example, a first fragment of a partially mapped read (e.g., identified as R1) will map to a first locus within the reference genome and the second fragment of the partially mapped read (e.g., identified as R1) will map to a second locus within the reference genome. A locus may be a mapped location for each of the read fragments on the reference genome. In some examples, the location may correspond to a known gene (e.g., with a gene name) and/or a known portion of a gene (e.g., known exon of a known gene). Similar to previous mappings/alignments, each fragment mapping may be within a threshold distance from the reference genome.

At 510, a candidate list of fusions can be generated based on an ordered pair of loci for the read fragments. For example, a subset of read fragments that each map to a loci on the reference genome may be selected. The candidate list of fusions may be a list of partially mapped reads where each fragment generated from the partially mapped read is selected for the subset. For example, the subset of read fragments may be analyzed such that fragments that contain matching keys (identifying the partially mapped read from which the fragment was generated) may be added to the candidate list. Based on the selection, read fragments from the same partially mapped read may be selected when each fragment from that partially mapped read is mapped to the reference genome.

The following data set illustrates an exemplary candidate fusion list, for instance as a database table. The entries may include each loci for the read fragments with accompanying data and a count for the particular loci combination.
[chr2:29446338-29446396, chr2:42491846-42491869] 1689
[chr4:39259398-39259419, chr6:170871271-170871321] 64
[chr2:29446338-29446396, chr2:42492057-42492089] 70
[chr12:128904278-128904299, chr6:170871271-170871321] 31
[chr19:55857939-55857959, chr1:156104280-156104320] 37
[chr2:29446327-29446396, chr2:42491846-42491869] 55
[chr1: 156104594-156104644, chrX:137308905-137308925] 70

At 512, the candidate fusions can be evaluated. For example, one or more filters may be applied to the candidate fusion list. In an embodiment, the number of reads with fragments mapping to the particular loci pair can be counted. For example, reads that generate fragments that map to the same (or substantially some) loci can be counted. An exemplary filter may comprise a threshold number of counted pairs.

In an exemplary embodiment, the loci can be annotated, such as with gene names and exon designations. For example, the reference genome may include accompanying metadata associated with the loci, such as gene names and exon designations. One or more filters may include at least one loci being annotated with a gene name, both loci being annotated with a gene name, at least one loci being annotated with an exon number, both loci being annotated with an exon number, and any suitable combination of filters. In some embodiments, a filter may include maximum distance from an (known) exon boundary (e.g., max number of bases or percentages of bases in the read fragment).

The following data set may illustrate a filtered and annotated candidate fusion list, for instance as a database table. The entries may include each loci for the read fragments with accompanying annotation data, if known, (e.g., gene name, exon number, sequence location, and the like), and a count for the particular loci combination.
[LMNA, SUV420H2E8] 45
[TBP, TMEM132C] 49
[TBP, WDR19] 91
[ALKE21, EML4E5] 1774
[ALKE21, EML4] 238
[LMNA, chrX: 137308905-137308925] 105

In various embodiments, the remaining candidate fusions after filtering and consolidating can be reported to a user. In some embodiments, one or more databases may be updated with data based on the filtered candidate fusion list. For example, a database that stores known gene fusions may be updated based on candidate fusion list. The database updates may be entries annotated with known fusion data (e.g., gene name, exon number, and other identifying information). In some examples, annotation data from the candidate fusion list may include previously unknown information about a known gene fusion (e.g., a distance from an exon boundary for the gene fusion, and the like), and the database may be updated with the previously unknown information. In some embodiments, one or more fusions from the candidate fusion list may not have been known (e.g., may not have been stored in a database of known gene fusions). In this example, the database of known gene fusions may be updated with the unknown gene fusion and any accompanying annotation data from the candidate fusion list.

In some embodiments, one or more data files may be generated such that a user may visualize the gene fusion. For example, the annotation data for the candidate gene fusions may be used to identify known genes, and a visualization of the gene fusion event based on the known genes may be generated. The visualization may be based on software executing on a computing device that interfaces with one or more displays such that a visualization (such as an animation) may be displayed.

In some embodiments, the breakpoints for candidate gene fusions may be unknown prior to sequencing. In some embodiments, a limited number of breakpoints may be known for the gene fusion (e.g., one of two known breakpoints). In some embodiments, the known information about the breakpoints may be limited (e.g., an exact location for the breakpoints may not be known). For instance, gene names may be known, but the precise coordinates for the breakpoints may be unknown. In these examples, the primer pool may be designed based on the limited knowledge for breakpoints. For instance, when one breakpoint is known a first primer may be designed based on the known breakpoint and a second primer may be a universal primer.

The exemplary workflow of FIG. 5 may include technical advantages over previous gene fusion detection methodologies. For example, some methodologies may require precise knowledge of fusion breakpoints in order to design a primer pool for sequencing. Other methodologies may not require such knowledge, but may be inefficient, slow, inaccurate, or require large amounts of processing due to excessive data that needs to be analyzed. Techniques according to various disclosed embodiments can provide an enhanced gene fusion detection methodology that does not require *a priori* knowledge of breakpoints, or at least can leverage only limited knowledge of breakpoints, and/or that efficiently detects gene fusions without excessive processing.

For example, one or more of identifying the subset of reads, soft clipping the subset of reads, generating the candidate fusion list based on matching keys, and/or filtering the candidate fusion list, as described herein, may reduce the number of false positives in the analysis, and subsequently reduce the amount of processing needed to detect the candidate gene fusions. In some embodiments, the filtering may reduce the size of the candidate fusion list by 90%, 80%, 70%, and the like. In other examples, for instance where data is known about the candidate gene fusions, the filtering may reduce the size of the list by a smaller percentage.

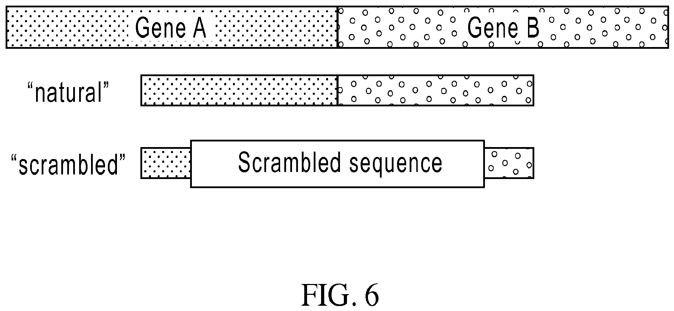

Figure 6 is a diagram showing an exemplary synthetic nucleotide controls. In various embodiments, the synthetic nucleotide control is a synthetic RNA control that can be used for fusion transcripts. The synthetic nucleotide can be spiked into the sample, prior to multiplex amplification. The "natural" spike-in sequence can be identical or substantially identical to the sequence of the fusion transcript. The "scrambled" spike-in sequence can be identical or substantially identical to the sequence of the fusion transcript only under the corresponding primer binding sites; the remaining internal sequence can be scrambled to distinguish it from the natural fusion transcript. For instance, the remaining internal sequence can be an arbitrary sequence of bases.

In various embodiments, a fusion assay may include a number of primer pairs that do not produce an amplicon. For example only a limited number of fusion species may be present in a positive sample, and some RNA samples may not have any of the targeted fusions species. Therefore, the data can produce "negative" evidence, in that a positive indicator is not triggered, and it may be challenging to determine if the primers are present and functional. The "scrambled" spike in sequence can provide a quality control mechanism for the multiplex amplification.

A scrambled spike in sequence ("scramblicon") can have the correct primer binding sequences for an RNA fusion amplicon, but between the two primer binding sites the sequence is scrambled such that the GC content and the length is maintained. These synthetic templates can clearly be distinguished from the native fusion species. If "native" templates were used to validate the RNA primers, it may be difficult to distinguish between the templates and the presence of the fusion in the sample. Additionally, a small amount of the "native" templates could contaminate samples and result in false positive results.

In various embodiments, the methods of the present teachings may be implemented in a software program and applications written in conventional programming languages such as C, C++, and the like.

While the present disclsoure sets forth various embodiments, it is not intended that the scope of the disclosure and claims be limited to such embodiments. On the contrary, those of ordinary skill in the art will appreciate that various alternatives, modifications, and equivalents are encompassed.

Further, in describing various embodiments, the specification may have presented a method and/or process as a particular sequence of steps. However, to the extent that the method or process does not rely on the particular order of steps set forth herein, the method or process should not be limited to the particular sequence or practice of steps described. As one of ordinary skill in the art would appreciate, other sequences of steps may be possible. Therefore, the particular order and inclusion of any of the steps set forth in the specification should not be construed as limitations on the claims. In addition, the claims directed to the method and/or process should not be limited to the performance of their steps in the order written, and one skilled in the art can readily appreciate that the sequences may be varied and still remain within the scope of the various embodiments.

The embodiments described herein, can be practiced with other computer system configurations including hand-held devices, microprocessor systems, microprocessor-based or programmable consumer electronics, minicomputers, mainframe computers and the like. The embodiments can also be practiced in distributing computing environments where tasks are performed by remote processing devices that are linked through a network.

It should also be understood that the embodiments described herein can employ various computer-implemented operations involving data stored in computer systems. These operations are those requiring physical manipulation of physical quantities. Usually, though not necessarily, these quantities take the form of electrical or magnetic signals capable of being stored, transferred, combined, compared, and otherwise manipulated. Further, the manipulations performed are often referred to in terms, such as producing, identifying, determining, or comparing.

Any of the operations that form part of the embodiments described herein are useful machine operations. The embodiments, described herein, also relate to a device or an apparatus for performing these operations. The systems and methods described herein can be specially constructed for the required purposes or it may be a general purpose computer selectively activated or configured by a computer program stored in the computer. In particular, various general purpose machines may be used with computer programs written in accordance with the teachings herein, or it may be more convenient to construct a more specialized apparatus to perform the required operations.

Certain embodiments can also be embodied as computer readable code on a computer readable medium. The computer readable medium is any data storage device that can store data, which can thereafter be read by a computer system. Examples of the computer readable medium include hard drives, network attached storage (NAS), read-only memory, random-access memory, CD-ROMs, CD-Rs, CD-RWs, magnetic tapes, and other optical and non-optical data storage devices. The computer readable medium can also be distributed over a network coupled computer systems so that the computer readable code is stored and executed in a distributed fashion.

## Claims

1. A method for detecting gene fusions comprising:
amplifying a nucleic acid sample in the presence of a primer pool to produce a plurality of amplicons;
wherein the primer pool includes a first set of primers corresponding to a 3' end of a first plurality of exons and a second set of primers corresponding to a 5' end of a second plurality of exons and wherein one set of the first and the second set of primers is designed based on a known breakpoint for a gene fusion and the other set of the first and the second set of primers comprises universal primers,
sequencing the amplicons by detecting a plurality of signals indicative of nucleotide incorporation events to generate a plurality of reads;
mapping the reads to a reference genome sequence based on alignments between the reads and the reference genome sequence;
identifying reads that partially map to the reference genome sequence for a subset of partially mapped reads;
generating read fragments for each partially mapped read of the subset by splitting the partially mapped read into a mapped portion and an unmapped portion;
aligning the read fragments generated from the subset of partially mapped reads to the reference genome sequence to determine loci on the reference genome for the mapped portions and the unmapped portions of the read fragments; and
identifying candidate fusions based on a combination of the loci of the reference genome determined in each of the partially mapped reads by the mapped portions and the unmapped portions of the read fragments.

2. The method of claim 1, further comprising filtering the candidate fusions based on a count for each particular loci combination of the mapped portion and the unmapped portion of the read fragments.

3. The method of claim 2, further comprising:
annotating the candidate fusions with known information corresponding to the loci comprising one or more of a gene name and an exon identification to form annotation data; and
filtering the candidate fusions based on the count for each particular loci combination of the mapped portion and the unmapped portion of the read fragments and the annotation data to form a filtered and annotated candidate fusion list.

4. The method of claim 1, further comprising updating a database of fusion genes with information based on the candidate fusions.

5. A system (300) for detecting gene fusions comprising:
a nucleic acid sequencing device (304) configured to:
sequence a plurality of amplicons by detecting a plurality of signals indicative of nucleotide incorporation events to generate a plurality of reads,
wherein the amplicons were produced by amplifying a nucleic acid sample in the presence of a primer pool;
wherein the primer pool includes a first set of primers corresponding to a 3' end of a first plurality of exons and a second set of primers corresponding to a 5' end of a second plurality of exons and wherein one set of the first and the second set of primers is designed based on a known breakpoint for a gene fusion and the other set of the first and the second set of primers comprises universal primers, and
an analytics computing device (302) comprising:
a mapping module (314) configured to map the reads to a reference genome sequence based on alignments between the reads and the reference genome sequence; and
a remapping engine (316) configured to:
(i) receive mapped reads from the mapping module (314) and identify reads that partially map to the reference genome for a subset of partially mapped reads;
(ii) generate read fragments for each partially mapped read of the subset by splitting the partially mapped read into a mapped portion and an unmapped portion;
(iii) align the read fragments generated from the subset of partially mapped reads to the reference genome to determine loci on the reference genome for the mapped portions and the unmapped portions; and
(iv) identify candidate fusions based on a combination of the loci of the reference genome determined in each of the partially mapped reads by the mapped portions and the unmapped portions of the read fragments.

6. The method of claim 1 or the system (300) of claim 5, wherein for the subset of partially mapped reads, a length of the mapped portion is less than or equal to 50% of a read length for the partially mapped read or less than or equal to 40% of the read length for the partially mapped read.

7. The method of claim 1 or the system (300) of claim 5, wherein the read fragments comprise a first fragment and a second fragment associated with each partially mapped read, wherein each first fragment and second fragment comprises a key associated with the partially mapped read from which the first and second fragments were generated.

8. The method of claim 7, further comprising selecting a subset of the read fragments that map to particular loci on the reference genome, wherein corresponding read fragments have the same key, wherein the subset of the read fragments are identified as candidate fusions.

9. The system (300) of claim 7, wherein the analytics computing device (302) is further configured to select a subset of the read fragments that map to particular loci on the reference genome, wherein corresponding read fragments have the same key, wherein the subset of the read fragments are identified as candidate fusions.

10. The system (300) of claim 5, wherein the analytics computing device (302) is further configured to filter the candidate fusions based on a count for each particular loci combination of the mapped portion and the unmapped portion of the read fragments.

11. The system (300) of claim10, wherein the analytics computing device (302) is further configured to:
annotate the candidate fusions with known information corresponding to the loci comprising one or more of a gene name and an exon identification to form annotation data; and
filter the candidate fusions based on the count for each particular loci combination of the mapped portion and the unmapped portion and the annotation data to form a filtered and annotated candidate fusion list.

12. The method of claim 1 or the system (300) of claim 5, wherein the candidate fusions are filtered based on at least one of an availability of a gene name for at least one of the read fragments, an availability of a gene name for both of the read fragments, an availability of an exon identification for at least one of the read fragments, and an availability of an exon identification for both of the read fragments.

13. The system of claim 5, wherein the analytics computing device (302) is further configured to update a database of fusion genes with information based on the candidate fusions.

## Patentansprüche

1. Verfahren zum Nachweisen von Genfusionen, umfassend:
Amplifizieren einer Nukleinsäureprobe in Gegenwart eines Primer-Pools, um mehrere Amplicons herzustellen;
wobei der Primer-Pool einen ersten Satz von Primern enthält, der einem 3'-Ende einer ersten Vielzahl von Exons entspricht, und einen zweiten Satz von Primern, der einem 5'-Ende einer zweiten Vielzahl von Exons entspricht, und wobei ein Satz des ersten und des zweiten Satzes von Primern basierend auf einer bekannten Bruchstelle für eine Genfusion gestaltet ist und der andere Satz des ersten und des zweiten Satzes von Primern universelle Primer umfasst,
Sequenzieren der Amplicons, durch Nachweisen einer Vielzahl von Signalen, die Nucleotid-Einbauereignisse anzeigen, um mehrere Reads zu generieren;
Kartieren der Reads auf eine Referenzgenomsequenz auf Basis von Alignments zwischen den Reads und der Referenzgenomsequenz;
Identifizieren von Reads, die teilweise auf der Referenzgenomsequenz kartiert sind, für eine Teilmenge von teilweise abgebildeten Reads;
Generieren von Readfragmenten für jeden teilweise kartierten Read der Teilmenge durch Aufteilen des teilweise kartierten Reads in einen kartierten Abschnitt und einen nicht kartierten Abschnitt;
Ausrichten der aus der Teilmenge von teilweise kartierten Reads generierten Readfragmente an der Referenzgenomsequenz, um Loci auf dem Referenzgenom für die kartierten Abschnitte und die nicht kartierten Abschnitte der Readfragmente zu bestimmen; und
Identifizieren von Kandidatenfusionen auf Basis einer Kombination der Loci des Referenzgenoms, die in jeder der teilweise kartierten Reads durch die kartierten Abschnitte und die nicht kartierten Abschnitte der Readfragmente bestimmt werden.

2. Verfahren nach Anspruch 1, das ferner das Filtern der Kandidatenfusionen auf Basis einer Zählung für jede bestimmte Loci-Kombination des kartierten Abschnitts und des nicht kartierten Abschnitts der Readfragmente umfasst.

3. Verfahren nach Anspruch 2, ferner umfassend:
Annotieren der Kandidatenfusionen mit bekannten Informationen in Entsprechung zu den Loci, die einen oder mehrere aus einem Gennamen und einer Exon-Identifikation umfassen, um Annotationsdaten zu bilden; und
Filtern der Kandidatenfusionen auf Basis der Zählung für jede bestimmte Loci-Kombination des kartierten Abschnitts und des nicht kartierten Abschnitts der Readfragmente und der Annotierungsdaten, um eine gefilterte und annotierte Kandidatenfusionsliste zu bilden.

4. Verfahren nach Anspruch 1, ferner umfassend das Aktualisieren einer Datenbank von Fusionsgenen mit Informationen auf Basis der Kandidatenfusionen.

5. System (300) zum Nachweisen von Genfusionen, umfassend:
eine Nukleinsäuresequenzierungsvorrichtung (304), die dazu konfiguriert ist:
mehrere Amplicons durch Nachweisen mehrerer Signale zu sequenzieren, die Nukleotid-Einbauereignisse anzeigen, um mehrere Reads zu erzeugen, wobei die Amplicons durch Amplifizieren einer Nukleinsäureprobe in Gegenwart eines Primer-Pools generiert wurden;
wobei der Primer-Pool einen ersten Satz von Primern enthält, der einem 3'-Ende einer ersten Vielzahl von Exons entspricht, und einen zweiten Satz von Primern, der einem 5'-Ende einer zweiten Vielzahl von Exons entspricht, und wobei ein Satz des ersten und des zweiten Satzes von Primern basierend auf einer bekannten Bruchstelle für eine Genfusion gestaltet ist und der andere Satz des ersten und des zweiten Satzes von Primern universelle Primer umfasst, und
eine Analytik-Rechenvorrichtung (302), umfassend:
ein Kartierungsmodul (314), das dazu konfiguriert ist, die Reads auf Basis von Alignments zwischen den Reads und der Referenzgenomsequenz auf einer Referenzgenomsequenz zu kartieren; und
eine Re-Kartierungssmaschine (316), die dazu konfiguriert ist:
(i) kartierte Reads vom Kartierungsmodul (314) zu erhalten und Reads zu identifizieren, die teilweise auf dem Referenzgenom kartiert sind für eine Teilmenge teilweiser kartierter Reads;
(ii) Readfragmente für jeden teilweise kartierten Read der Teilmenge zu generieren durch Aufteilen des teilweise kartierten Reads in einen kartierten Abschnitt und einen nicht kartierten Abschnitt;
(iii) die aus der Teilmenge von teilweise kartierten Reads generierten Readfragmente an dem Referenzgenom auszurichten, um Loci auf dem Referenzgenom für die kartierten Abschnitte und die nicht kartierten Abschnitte zu bestimmen; und
(iv) Kandidatenfusionen auf Basis einer Kombination der Loci des Referenzgenoms, die in jedem der teilweise kartierten Reads bestimmt wurde, durch die kartierten Abschnitte und die nicht kartierten Abschnitte der Readfragmente zu identifizieren.

6. Verfahren nach Anspruch 1 oder System (300) nach Anspruch 5, wobei für die Teilmenge teilweise kartierter Reads eine Länge des kartierten Abschnitts kleiner oder gleich 50 % einer Readlänge für den teilweise kartierten Read oder kleiner oder gleich 40 % der Readlänge für den teilweise kartierten Read ist.

7. Verfahren nach Anspruch 1 oder System (300) nach Anspruch 5, wobei die Readfragmente ein erstes Fragment und ein zweites Fragment umfassen, die jedem teilweise kartierten Read zugeordnet sind, wobei jedes erste Fragment und zweite Fragment einen Schlüssel umfasst, der dem teilweise kartierten Read zugeordnet ist, aus dem das erste und das zweite Fragment generiert wurden.

8. Verfahren nach Anspruch 7, ferner umfassend das Auswählen einer Teilmenge der Readfragmente, die auf bestimmten Loci auf dem Referenzgenom kartiert sind, wobei entsprechende Readfragmente denselben Schlüssel aufweisen, wobei die Teilmenge der Readfragmente als Kandidatenfusionen identifiziert wird.

9. System (300) nach Anspruch 7, wobei die Analytik-Rechenvorrichtung (302) ferner dazu konfiguriert ist, eine Teilmenge der Readfragmente auszuwählen, die auf bestimmten Loci auf dem Referenzgenom kartiert sind, wobei entsprechende Readragmente denselben Schlüssel aufweisen, wobei die Teilmenge der Readfragmente als Kandidatenfusionen identifiziert wird.

10. System (300) nach Anspruch 5, wobei die Analytik-Rechenvorrichtung (302) ferner dazu konfiguriert ist, die Kandidatenfusionen auf Basis einer Zählung für jede bestimmte Loci-Kombination des kartierten Abschnitts und des nicht kartierten Abschnitts der Readfragmente zu filtern.

11. System (300) nach Anspruch 10, wobei die Analytik-Rechenvorrichtung (302) ferner dazu konfiguriert ist:
Kandidatenfusionen mit bekannten Informationen entsprechend den Loci zu annotieren, die einen oder mehrere aus einem Gennamen und einer Exon-Identifikation umfassen, um Annotierungsdaten zu bilden; und
die Kandidatenfusionen auf Basis der Zählung für jede bestimmte Loci-Kombination des kartierten Abschnitts und des nicht kartierten Abschnitts und der Annotierungsdaten zu filtern, um eine gefilterte und annotierte Kandidatenfusionsliste zu bilden.

12. Verfahren nach Anspruch 1 oder System (300) nach Anspruch 5, wobei die Kandidatenfusionen auf Basis mindestens eines aus einer Verfügbarkeit eines Gennamens für mindestens eines der Readfragmente, einer Verfügbarkeit eines Gennamens für beide Readfragmente, einer Verfügbarkeit einer Exon-Identifikation für mindestens eines der Readfragmente und einer Verfügbarkeit einer Exon-Identifikation für beide Readfragmente gefiltert werden.

13. System nach Anspruch 5, wobei die Analytik-Rechenvorrichtung (302) ferner dazu konfiguriert ist, eine Datenbank von Fusionsgenen mit Informationen auf Basis der Kandidatenfusionen zu aktualisieren.

## Revendications

1. Procédé de détection de fusions de gènes comprenant :
l'amplification d'un échantillon d'acide nucléique en présence d'une masse commune d'amorces afin de produire une pluralité d'amplicons ;
la masse commune d'amorces comprenant un premier ensemble d'amorces correspondant à une extrémité 3' d'une première pluralité d'exons et un second ensemble d'amorces correspondant à une extrémité 5' d'une seconde pluralité d'exons, et un ensemble du premier et du second ensemble d'amorces étant conçu en fonction d'un point de rupture connu pour une fusion de gènes et l'autre ensemble du premier et du second ensemble d'amorces comprenant des amorces universelles, le séquençage des amplicons par la détection d'une pluralité de signaux indiquant des événements d'incorporation de nucléotides afin de produire une pluralité de lectures ;
le mappage des lectures avec une séquence de génome de référence en fonction des alignements entre les lectures et la séquence de génome de référence ;
l'identification des lectures qui mappent partiellement la séquence de génome de référence pour un sous-ensemble de lectures partiellement mappées ;
la production de fragments de lecture pour chaque lecture partiellement mappée du sous-ensemble par la division de la lecture partiellement mappée en une partie mappée et une partie non mappée ;
l'alignement des fragments de lecture produits provenant du sous-ensemble de lecture partiellement mappée avec la séquence de génome de référence afin de déterminer des loci sur le génome de référence pour les parties mappées et les parties non mappées des fragments de lecture ; et
l'identification de fusions de candidats en fonction d'une combinaison des loci du génome de référence déterminés dans chacune des lectures partiellement mappées par les parties mappées et les parties non mappées des fragments de lecture.

2. Procédé selon la revendication 1, comprenant en outre la filtration des fusions de candidats en fonction d'un comptage pour chaque combinaison particulière de loci de la partie mappée et de la partie mappée des fragments de lecture.

3. Procédé selon la revendication 2, comprenant en outre :
la notation des fusions de candidats avec des informations connues correspondant aux loci comprenant au moins l'un parmi un nom de gène et une identification d'exon afin de former des données d'annotation ;
et la filtration des fusions de candidats en fonction du comptage pour chaque combinaison particulière de loci de la partie mappée et de la partie non mappée des fragments de lecture, et des données d'annotation afin de former une liste de fusion de candidats filtrée et annotée.

4. Procédé selon la revendication 1, comprenant en outre la mise à jour d'une base de données de gènes de fusions avec des informations basées sur les fusions de candidats.

5. Système (300) de détection de fusions de gènes comprenant :
un dispositif de séquençage d'acide nucléique (304) conçu pour :
séquencer une pluralité d'amplicons par la détection d'une pluralité de signaux indiquant les événements d'incorporation de nucléotides afin de produire une pluralité de lectures, les amplicons étant produits par l'amplification d'un échantillon d'acide nucléique en présence d'une masse commune d'amorces ;
la masse commune d'amorces comprenant un premier ensemble d'amorces correspondant à une extrémité 3' d'une première pluralité d'exons et un second ensemble d'amorces correspondant à une extrémité 5' d'une seconde pluralité d'exons et un ensemble du premier et du second ensemble d'amorces étant conçu en fonction d'un point de rupture connu pour une fusion de gènes et l'autre ensemble du premier et du second ensemble d'amorces comprenant des amorces universelles, et un dispositif de calcul analytique (302) comprenant :
un module de mappage (314) conçu pour mapper les lectures dans une séquence de génome de référence en fonction d'alignements entre les lectures et la séquence de génome de référence ; et
un moteur de remappage (316) conçu pour :
(i) recevoir des lectures mappées provenant du module de mappage (314) et identifier les lectures qui mappent partiellement le génome de référence pour un sous-ensemble de lectures partiellement mappées ;
(ii) produire des fragments de lecture pour chaque lecture partiellement mappée du sous-ensemble par la division de la lecture partiellement mappée en une partie mappée et en une partie non mappée ;
(iii) aligner les fragments de lecture produits provenant du sous-ensemble de lectures partiellement mappées du génome de référence afin de déterminer des loci sur le génome de référence pour les parties mappées et les parties non mappées ; et
(iv) identifier des fusions de candidats en fonction d'une combinaison des loci du génome de référence déterminé dans chacune des lectures partiellement mappées par les parties mappées et les parties non mappées des fragments de lecture.

6. Procédé selon la revendication 1 ou système (300) selon la revendication 5, dans lequel pour le sous-ensemble de lectures partiellement mappées, une longueur de la partie mappée est inférieure ou égale à 50 % de la longueur de lecture pour la lecture partiellement mappée, ou inférieure ou égale à 40 % de la longueur de lecture pour la lecture partiellement mappée.

7. Procédé selon la revendication 1 ou système (300) selon la revendication 5, dans lequel les fragments de lecture comprennent un premier fragment et un second fragment associés à chaque lecture partiellement mappée, chaque premier fragment et second fragment comprenant une clé associée à la lecture partiellement mappée à partir de laquelle les premier et second fragments ont été produits.

8. Procédé selon la revendication 7, comprenant en outre la sélection d'un sous-ensemble des fragments de lecture qui mappent des loci particuliers sur le génome de référence, des fragments correspondants de lecture ayant la même clé, le sous-ensemble des fragments de lectures étant identifiés comme des fusions de candidats.

9. Système (300) selon la revendication 7, dans lequel le dispositif de calcul analytique (302) étant conçu pour choisir un sous-ensemble des fragments de lecture qui mappent des loci particuliers sur le génome de référence, des fragments correspondants de lecture ayant la même clé, le sous-ensemble des fragments de lecture étant identifiés comme des fusions de candidats.

10. Système (300) selon la revendication 5, dans lequel le dispositif de calcul analytique (302) est en outre conçu pour filtrer les fusions de candidats en fonction d'un comptage pour chaque combinaison particulière de loci de la partie mappée et de la partie non mappée des fragments de lecture.

11. Système (300) selon la revendication 10, dans lequel le dispositif de calcul analytique (302) est en outre conçu pour :
annoter les fusions de candidats avec des informations connues correspondant aux loci comprenant l'au moins un parmi un nom de gène et une identification d'exon afin de former des données d'annotation ; et
filtrer les fusions de candidats en fonction du comptage pour chaque combinaison particulière de loci de la partie mappée et de la partie non mappée, et les données d'annotation afin de former une liste de fusion de candidats filtrée et annotée.

12. Procédé selon la revendication 1 ou système (300) selon la revendication 5, dans lequel les fusions de candidats sont filtrées en fonction de l'au moins une disponibilité d'un nom de gène pour au moins un parmi les fragments de lecture, une disponibilité d'un nom de gène pour les deux fragments de lecture, une disponibilité d'une identification d'exon pour au moins un parmi les fragments de lecture et une disponibilité d'une identification d'exon pour les deux fragments de lecture.

13. Système selon la revendication 5, dans lequel le dispositif de calcul analytique (302) est en outre conçu pour mettre à jour une base de données de gènes de fusions avec des informations basées sur les fusions de candidats.
